# EUROPEAN PATENT SPECIFICATION

(11) **EP 3 085 372 B1**
(45) Date of publication and mention of the grant of the patent: **28.07.2021**
(21) Application number: 13876084.8
(22) Date of filing: 20.12.2013
(51) Int. Cl.: A61K 31/4415, A61K 31/519, A61K 33/06, A61K 33/10, A61P 35/00

(54) **COMBINATION OF PYRIDOXINE, FOLIC ACID AND MAGNESIUM IONS FOR TREATING CANCER**
KOMBINATION AUS PYRIDOXIN, FOLSÄURE UND MAGNESIUMIONEN ZUR BEHANDLUNG VON KREBS
COMBINAISON DE PYRIDOXINE, D'ACIDE FOLIQUE ET D'IONS MAGNÉSIUM POUR TRAITER LE CANCER

(43) Date of publication of application: 26.10.2016
(73) Proprietor: Vergara Campillo, Ramiro Moises, BOGOTA D.C. (CO)
(72) Inventor: Vergara Campillo, Ramiro Moises, BOGOTA D.C. (CO)
(74) Representative: Del Valle Valiente, Sonia
(86) International application number: PCT/IB2013/002861
(87) International publication number: WO 2015/107375

(56) References cited:
- US-A1- 2006 024 409
- US-B2- 9 572 810

## Description

### STATE OF THE ART

Until now, medical science approaches the pathology of malign and benign tumours based on two main principles, namely the external noxa and the location of the disease. The principle of the external noxa establishes that any disease of the human body is generated by an external agent which penetrates in the organism through a solution of continuity in the skin or the mucosae covering said skin, which then turns into an entry point for the pathogen agent that, once inside, produces the disease merely by its pathogenic property, irrespective on the conditions of the host.

The principle of the location of the disease establishes that diseases only compromise one organ or system forming part of the body, and its activity is focused on finding this location and fighting the pathology in the location found, in the belief that by eliminating the diseased organ a healthy condition can be restored.

Current medical science has a warlike attitude towards pathology, and it therefore proposes fighting the disease by means of destructive procedures for attacking the diseased organ; thus, three procedures are employed: surgery, radiotherapy, and chemotherapy. The object of surgery is to extirpate the diseased organ, or a large portion thereof, including the tumour and large masses of neighbouring tissue. Radiotherapy is based on radioactive wave generating sources attempting to destroy the tumour; however, radioactive waves inevitable also go through healthy tissue at the same time, thus destroying it. Chemotherapy is based on chemical substances being highly toxic for the cellular membrane attempting to destroy the tumour; however, once these substances are in the blood circulation, they also attack healthy tissue and organs. The active compounds referred to in the present invention, pyridoxine, folic acid and magnesium ions in the form of chloride salt, are not found together but separately, as disclosed in patent documents WO 2008048085 A1, WO 2001095887 A1 and WO 2011041920 A8. US 9,572,810 B discloses combinations of the three claimed compounds for treating cancer, but also comprises sodium ascorbate, magnesium chloride 2 H2o, 2 di-methyl amino ethanol HCI thiamine, riboflavin, nicotinamide, pyridoxine, calcium pantothenate, cyanocobalamin, and folic acid, and one or more ingredients selected from the group consisting of vitamins, salts, acids, amino acids or salts thereof, vitamers, one or more anaesthetic substance, stabilized oxidative species, heparin, and lipoic acid and salts or mixtures thereof. The present invention differs in that a ternary combination is claimed, which is the sole active ingredient. The medication disclosed below solves the cancer problem without producing the side effects.

### OBJECTS

One of the main objects of this new medicament is that in treating cancer, when restoring the normal functioning of the tissues, the tumours disappear; and it does not only solve present tumours, but it also prevents and cures any possible metastases, and it also prevents new tumours with no side effects. Then, the invention refers to a ternary combination of pyridoxine, folic acid and magnesium ion as a chloride or carbonate, for use as the sole active ingredient in the treatment of cancer, wherein normal behaviour of tissue is restored. In addition, the invention refers to an injectable for intravenous administration, a syrup or a tablet comprising the ternary combination of pyridoxine, folic acid and magnesium ions as chloride or carbonate as the sole active ingredient for use in the treatment of cancer, wherein normal behaviour of tissue is restored.

Another important object is that the medicament has no negative or undesirable effect on the patient; on the contrary, it contributes to an increase of vitality and to an improvement in the quality of life from the moment the treatment begins.

Another object is that pyridoxine, folic acid and magnesium ions in the form of chloride salt and/or carbonate, by being associated and administered simultaneously in their exact proportion, cover a 360-degree functional spectrum in regulating the synthesis of the main 4 neurotransmitters in the brain.

### DETAILED DESCRIPTION OF THE INVENTION

The present invention refers to a ternary combination of pyridoxine, folic acid and magnesium ions as chloride or carbonate, for use as the sole active ingredient in the treatment of cancer, wherein normal behaviour of tissue is restored, wherein the combination is to be administered as intravenous administration in one or more doses per day, the amount of pyridoxine to be administered per day is 1g, the amount of folic acid to be administered per day is 20 mg, and the amount of magnesium to be administered per day is 500 milligrams. The scope of the present invention is defined in the claims; any other disclosure in the present description not protected by the appended claims, regardless of whether it is indicated as being preferred or exemplified, is provided for referential purposes, only.

The medicament is formed by the combination of active ingredients comprising a combination of pyridoxine, folic acid and magnesium ions, and it also contains two vitamins and one trace element which were traditionally prescribed as coadjuvants in the treatments of diseases where the general state and the vitality of the patient are at risk, but which were not a mandatory prescription; on the contrary, they were traditionally added to the main and basic treatments, which were believed to provide the expected effect, depending on the gut feelings of the doctor in charge. Some healthcare professionals advise against their use because they consider it a loss of resources.

This composition is the result of a research work using a new research method, as a result of which it was found that the disease has a special neuro-physical mechanism and a psychological cause; this discovery lead to the approach disclosed in this new therapeutic intervention. The method used was called New Scientific Clinical Applied Research (NICCA, Nueva Investigación Científica Clínica Aplicada); it consists on a clinical and scientific observation enhanced with information from psychoanalysis, neuroscience and neurolinguistic programming.

This new tool, the normal pathologic behaviour of the organism is seen as if magnified by a magnifying glass; it is magnified and, at the same time, the intervention of the brain for managing the peripheral organs and tissues in their normal state is visible; and also how the brain has the necessary hardware and software for inducing the pathologic state and also for, after a correct therapeutic intervention, restoring it to normal.

NICCA allows for the design of therapeutic interventions based on medicaments capable of curing diseases which, until now, have been considered incurable; therapeutic interventions such as the present composition, which covers a 360 degree spectrum in the brain, in the regulation of the synthesis of the four main neurotransmitters of the brain, starting from the neurotransmitters, which is the main metabolic route for the synthesis of Gaba from glutamic acid, which is carried out by the action of Glutamic Acid Decarboxylase (GAD), this requiring as co-ferments pyridoxal phosphate, which is a salt derived from pyridoxine, plus the phosphoric acid and it also requires magnesium ions (Mg++).

Similarly, the synthesis of glycine taking place from serine, by the action of the serine-hydroxyl-methyl-transferase (SHMT) enzyme, also requires as a co-ferment the pyridoxal phosphate in addition to magnesium ions (Mg++) and folic acid.

The synthesis of glutamine, glutamic acid and the Gaba is carried out by means of a close cooperation between the neuron and the glia cell, such that both of them together make the Gabashunt. This biochemical process involves, on one hand, Gaba-Transaminase (Gaba-T) and Succinic SemiAldehyde DeHydrogenase (SSADH) enzymes, which operate only within the mitochondria and, on the other hand, glutaminase or Glutaminic Acid Decarboxilase (GAD) and the Glutamine Synthetase (Glu-Synth), both being cytoplasmic enzymes. On the other hand, GAD exists only within the neuron and Glu-Synth exists only within the glia. Thus, a cooperation between these two cells is necessary for the metabolism of the two most numerous neurotransmitters in the Central Nervous System (SNC).

The Gaba released in the synaptic slit is taken by Gaba-T and forms succinic semialdehyde, which then turns into succinic acid and enters the Krebs cycle again. The glia, in turn, forms glutamic acid, which is the precursor of the Gaba, from alfa oxoglutaric acid by the action of the asparto-amino-transferase; however, this metabolic route stops here and no Gaba is formed, because glia's cell lacks GAD, having glutamine-synthetase instead, which acts on the glutamic acid to form glutamine; the glutamine, in turn, passes to the neuron, where it forms glutamic acid by the action of the glutaminase. The glutamic acid source in the neuron is glucose, from which alpha oxoglutaric acid is derived; the alpha oxoglutaric acid, in turn, is transaminated by the aspartate-amino-transferase for forming glutamic acid, which is then transformed into Gaba by the decarboxylase in the glutamic acid (GAD); degradation of Gaba takes place by means of the Gaba transaminase for forming succinic semialdehyde. However, this reaction takes place only if the acceptor of the amine group is again the alpha oxoglutaric acid, which then gives rise to a new glutamic acid molecule, which is a precursor of Gaba.

This proves the importance of Gaba for the nervous system, as it confers this chemical protection to said molecule for ensuring a permanent provision of Gaba to the neuron; this means that a Gaba molecule can only be metabolically destroyed if, at the same time, a precursor of Gaba taking its place is formed.

An intervention by means of a combination in the necessary proportions of the substances proposed herein, known as ECOSAN, restores the normal behavior of tissues; it is useful for treating both benignant and malignant tumors, since NICCA allowed for the discovery of the efficient causality of the mechanism of both types of tumors. There is no need to use surgery, radiotherapy and chemotherapy.

The known methods for treating tumors are known to generate very destructive side effects. The use of surgery causes the patients to be extensively mutilated and their functions diminished; radiotherapy causes injuries such as ulcerations that never heal again; chemotherapy causes generalized injuries in the nervous system and in the tissues generating blood cells, among others.

Proposed treatment- Using NICCA as the research method, it was determined that even though something must indeed enter the organism for generating the disease, this something is not a microscopic agent but it is always information; information makes up the unconscious mind driving the brain, and the body expresses the changes based on the information received by the sense organs, which act as external agents for the brain.

Note that the mind is made of information; the information is made up by small movies grouped in sectors according to related information content and they are autonomous, such that they are not dependent on will; they react to new information content entering through the sense organs and produce effects which may be destructive.

NICCA also proved that while the symptoms and clinical signs are perceived by the patient in a limited portion of his/her body, this location is just a place chosen by the unconscious mind to express its message; however, the information acts in the center, in the brain, where the information contents are all related to each other. This means that when a symptom appears, the decision for sending the message made by all levels of the system was unanimous; a location is chosen that symbolizes something in connection with the information generating the disease.

Furthermore, NICCA also proved that both the unconscious mind and the brain can be completely versatile, that is, they can do one thing and the opposite; this situation requires a therapeutic intervention based on the principles disclosed herein, and thus ECOSAN was created.

In view of all this, the treatment proposed in the present patent application has the object of increasing the vitality of the host, that is, the person suffering the disease, and is directed to deprogramming the destructive impulses that appeared in the person with no undesirable side effect; the treatment does not have any contraindications and is not incompatible with other medicinal chemical substances.

The ECOSAN composition is made by three active ingredients:
a. Pyridoxine
b. Folic acid
c. Magnesium ions in the form of carbonate or chloride salt.

This composition can be administered orally, but in order to effectively treat tumors an intravenous parenteral administration is required. The necessary concentrations are also novel, because currently known dietetics considers that a normal adult must take 3 mg of pyridoxine with the meals a day; however, in the case of tumors, doses consisting of a gram intravenously administered are necessary; depending on the condition of the patient, more than a single dose in 24 hours can be necessary. As to folic acid, 100 micrograms with the meals a day are recommended for an adult; however, for treating tumors, doses consisting of 20 milligrams intravenously administered are necessary; depending on the condition of the patient, more than a single dose in 24 hours can be necessary. As to magnesium ions, 20 milligrams with the meals a day are recommended for an adult; however, for treating tumors, doses consisting on 500 milligrams intravenously administered are necessary; depending on the condition of the patient, more than a single dose in 24 hours can be necessary.

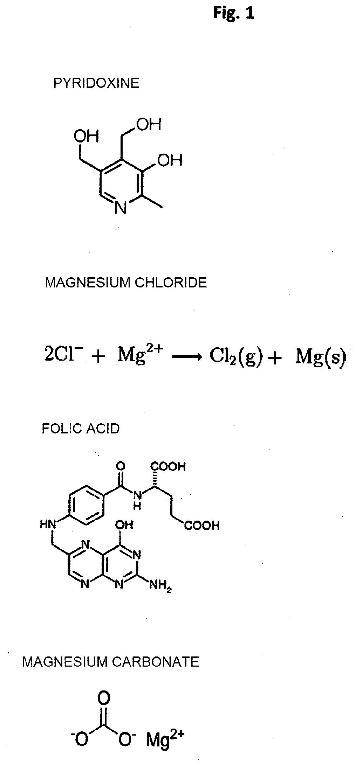

## Claims

1. A ternary combination of pyridoxine, folic acid and magnesium ions as chloride or carbonate, for use as the sole active ingredient in the treatment of cancer, wherein normal behavior of tissue is restored, wherein the combination is to be administered as intravenous administration in one or more doses per day, the amount of pyridoxine to be administered per day is 1 g, the amount of folic acid to be administered per day is 20 mg, and the amount of magnesium to be administered per day is 500 mg.

2. The ternary combination for use according to claim 1 wherein:
a) the pyridoxine is at 66%, folic acid at 9% and magnesium chloride at 25%, or
b) the pyridoxine is at 65%, folic acid at 8% and magnesium chloride at 27%, or
c) the pyridoxine is at 65%, folic acid at 9% and magnesium chloride at 26%, or
d) the pyridoxine is at 65% folic acid at 10% and magnesium chloride at 25%, or
e) the pyridoxine is at 64% folic acid at 9% and magnesium chloride at 27%, or
f) the pyridoxine is at 63% folic acid at 8% and magnesium chloride at 29%, or
g) the pyridoxine is at 62% folic acid 7% and magnesium chloride 31%, or
h) the pyridoxine is at 61% folic acid 6% and magnesium chloride 33%, or
i) the pyridoxine is at 60% folic acid 5% and magnesium chloride 35%, or
j) the pyridoxine is at 90% folic acid 2% and magnesium chloride 8%, or
k) the pyridoxine is at 85% folic acid 1% and magnesium chloride 14%.

3. An injectable for intravenous administration comprising the ternary combination of pyridoxine, folic acid and magnesium ions as chloride or carbonate as the sole active ingredient for use according to claim 1 or 2.

## Patentansprüche

1. Ternäre Kombination aus Pyridoxin, Folsäure und Magnesiumionen als Chlorid oder Carbonat zur Verwendung als einziger Wirkstoff bei der Behandlung von Krebs, wobei normales Verhalten von Gewebe wiederhergestellt wird, wobei die Kombination als intravenöse Verabreichung in einer oder mehreren Dosen pro Tag zu verabreichen ist, die Menge an Pyridoxin, die pro Tag zu verabreichen ist, 1 g beträgt, die Menge an Folsäure, die pro Tag zu verabreichen ist, 20 mg beträgt und die Menge an Magnesium, die pro Tag zu verabreichen ist, 500 mg beträgt

2. Ternäre Kombination zur Verwendung nach Anspruch 1, wobei:
a) das Pyridoxin bei 66 %, Folsäure bei 9 % und Magnesiumchlorid bei 25 % liegt oder
b) das Pyridoxin bei 65 %, Folsäure bei 8 % und Magnesiumchlorid bei 27 % liegt oder
c) das Pyridoxin bei 65 %, Folsäure bei 9 % und Magnesiumchlorid bei 26 % liegt oder
d) das Pyridoxin bei 65 %, Folsäure bei 10 % und Magnesiumchlorid bei 25 % liegt oder
e) das Pyridoxin bei 64 %, Folsäure bei 9 % und Magnesiumchlorid bei 27 % liegt oder
f) das Pyridoxin bei 63 %, Folsäure bei 8 % und Magnesiumchlorid bei 29 % liegt oder
g) das Pyridoxin bei 62 %, Folsäure bei 7 % und Magnesiumchlorid bei 31 % liegt oder
h) das Pyridoxin bei 61 %, Folsäure bei 6 % und Magnesiumchlorid bei 33 % liegt oder
i) das Pyridoxin bei 60 %, Folsäure bei 5 % und Magnesiumchlorid bei 35 % liegt oder
j) das Pyridoxin bei 90 %, Folsäure bei 2 % und Magnesiumchlorid bei 8 % liegt oder
k) das Pyridoxin bei 85 %, Folsäure bei 1 % und Magnesiumchlorid bei 14 % liegt.

3. Injizierbares Mittel zur intravenösen Verabreichung, umfassend die ternäre Kombination aus Pyridoxin, Folsäure und Magnesiumionen als Chlorid oder Carbonat als einzigen Wirkstoff zur Verwendung nach Anspruch 1 oder 2.

## Revendications

1. Combinaison ternaire de pyridoxine, d'acide folique et d'ions magnésium en tant que chlorure ou carbonate, pour une utilisation en guise de seul ingrédient actif dans le traitement d'un cancer, dans laquelle un comportement normal d'un tissu est restauré, dans laquelle la combinaison doit être administrée en tant qu'administration intraveineuse en une ou plusieurs doses par jour, la quantité de pyridoxine à administrer par jour est de 1 g, la quantité d'acide folique à administrer par jour est de 20 mg, et la quantité de magnésium à administrer par jour est de 500 mg

2. Combinaison ternaire pour utilisation selon la revendication 1 dans laquelle :
a) la pyridoxine est à 66 %, l'acide folique à 9 % et le chlorure de magnésium à 25 %, ou
b) la pyridoxine est à 65 %, l'acide folique à 8 % et le chlorure de magnésium à 27 %, ou
c) la pyridoxine est à 65 %, l'acide folique à 9 % et le chlorure de magnésium à 26 %, ou
d) la pyridoxine est à 65 % l'acide folique à 10 % et le chlorure de magnésium à 25 %, ou
e) la pyridoxine est à 64 % l'acide folique à 9 % et le chlorure de magnésium à 27 %, ou
f) la pyridoxine est à 63 % l'acide folique à 8 % et le chlorure de magnésium à 29 %, ou
g) la pyridoxine est à 62 % l'acide folique 7 % et le chlorure de magnésium 31 %, ou
h) la pyridoxine est à 61 % l'acide folique 6 % et le chlorure de magnésium 33 %, ou
i) la pyridoxine est à 60 % l'acide folique 5 % et le chlorure de magnésium 35 %, ou
j) la pyridoxine est à 90 % l'acide folique 2 % et le chlorure de magnésium 8 %, ou
k) la pyridoxine est à 85 % l'acide folique 1 % et le chlorure de magnésium 14%.

3. Injectable pour administration intraveineuse comprenant la combinaison ternaire de pyridoxine, d'acide folique et d'ions magnésium en tant que chlorure ou carbonate en guise de seul ingrédient actif pour utilisation selon la revendication 1 ou 2.
